# EUROPÄISCHE PATENTSCHRIFT

(11) **EP 0 829 240 B1**
(45) Veröffentlichungstag und Bekanntmachung des Hinweises auf die Patenterteilung: **03.03.1999**
(21) Anmeldenummer: 97115051.1
(22) Anmeldetag: 29.08.1997
(51) Int. Cl.: A61B 17/80

(54) **Knochenplatte**
Bone plate
Plaque d'ostéosynthèse

(30) Priorität: 17.09.1996 DE 19637938
(43) Veröffentlichungstag der Anmeldung: 18.03.1998
(73) Patentinhaber: Harms, Jürgen, Prof. Dr., 76337 Waldbronn (DE); Biedermann, Lutz, 78048 Villingen-Schwenningen (DE)
(72) Erfinder: Harms, Jürgen, Prof. Dr., 76337 Waldbronn (DE); Biedermann, Lutz, 78048 Villingen-Schwenningen (DE)
(74) Vertreter: Prüfer, Lutz H., Dipl.-Phys.

(56) Entgegenhaltungen:
- WO-A-96/02198
- DE-C- 4 007 306
- FR-A- 1 239 266
- FR-A- 2 689 750
- GB-A- 627 580

## Beschreibung

Die Erfindung betrifft eine Knochenplatte nach dem Oberbegriff des Patentanspruches 1.

Derartige Knochenplatten werden insbesondere in der Wirbelsäulenchirugie eingesetzt. Dabei werden verschiedene Längen der Knochenplatten mit verschiedenen Abständen der jeweiligen Knochenschrauben benötigt. Der Operateur hat Zu diesem Zweck einen Satz verschiedener Größen solcher Platten auf Vorrat. Schon diese Lagerhaltung ist nachteilig. Darüber hinaus erfordert die Operation oftmals eine Ausrichtung der miteinander zu verbindenden Teile, so daß bei einer Operation nacheinander Platten verschiedener Längen angewendet werden müssen.

Aufgabe der Erfindung ist es, eine Knochenplatte der eingangs beschriebenen Art zu schaffen, die die oben beschriebenen Nachteile beseitigt.

Diese Aufgabe wird durch die in Patentanspruch 1 gekennzeichnete Knochenplatte gelöst. Weiterbildungen der Erfindung sind in den Unteransprüchen gekennzeichnet.

Mit der erfindungsgemäßen Knochenplatte erübrigt sich nicht nur die nachteilige Lagerhaltung, sondern es ist auch möglich, durch Verändern der Relativstellung der beiden Abschnitte zueinander eine Distraktion bzw. Kontraktion der zu verbindenden Abschnitte vorzunehmen und jeweils eine Zwischenarretierung vorzunehmen, ohne daß die Verankerungsschrauben dazu erst gelöst bzw. die Knochenplatten nicht ausgetauscht werden müßten.

Weitere Merkmale und Zweckmäßigkeiten ergeben sich aus der Beschreibung eines Ausführungsbeispieles anhand der Figuren.

Von den Figuren zeigen:
- Fig. 1: eine Draufsicht auf die auseinandergenommenen beiden Abschnitte der Knochenplatte;
- Fig. 2: die Knochenplatte eingestellt auf die minimale Länge;
- Fig. 3: die gleiche Knochenplatte eingestellt auf die maximale Länge;
- Fig. 4: eine Seitenansicht einer Verankerungsschraube;
- Fig. 5: eine Seitenansicht einer Feststellschraube;
- Fig. 6: eine Schnittdarstellung durch ein Fixierelement;
- Fig. 7: eine Seitenansicht der Knochenplatte mit eingesetzten Verankerungsschrauben und Fixierelement in vergrößertem Maßstab;
- Fig. 8: eine Detaildarstellung aus Fig. 7 in weiter vergrößertem Maßstab;
- Fig. 9: einen Schnitt durch die Knochenplatte; und
- Fig. 10: einen Schnitt durch ein Sicherungselement.

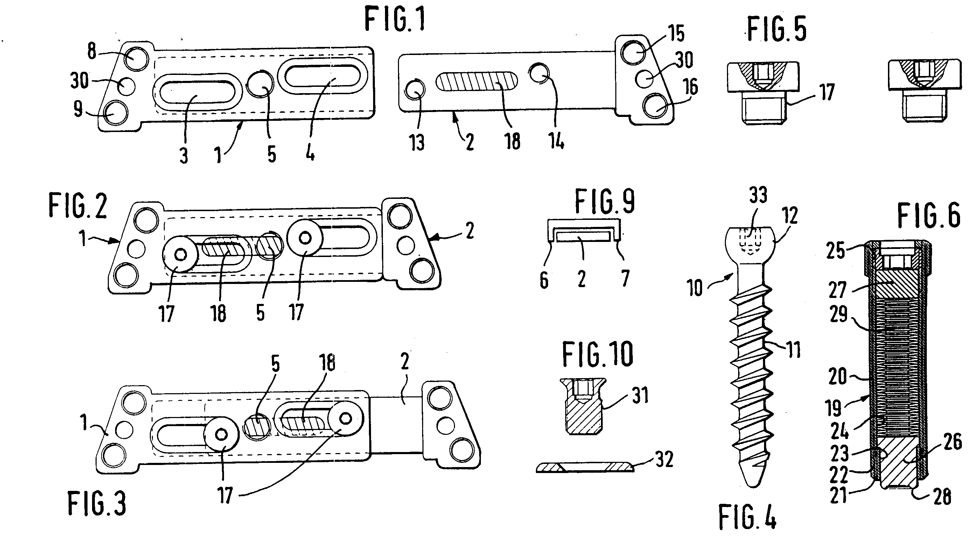

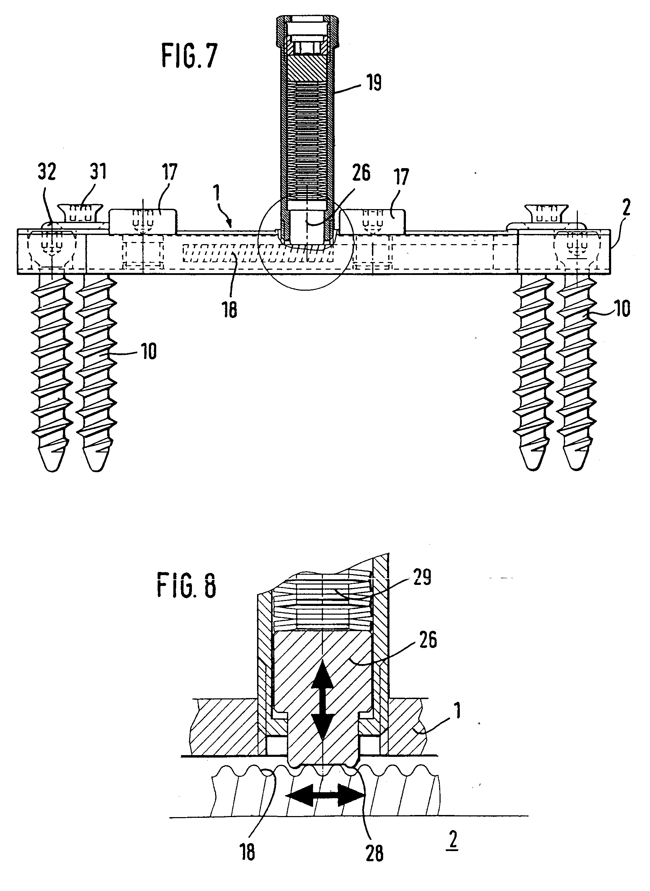

Die Knochenplatte wird zunächst anhand der Figuren 1 bis 3 beschrieben.

Die Knochenplatte weist einen ersten Abschnitt 1 und einen zweiten Abschnitt 2 auf. Der erste Abschnitt 1 besitzt die Form einer langgestreckten rechteckigen Platte mit einem ersten Langloch 3, welches sich parallel zur Längsachse und zu einem Seitenrand hin gegen die Mitte versetzt erstreckt. Ferner ist ein entsprechendes zweites Langloch 4 vorgesehen, welches sich parallel zum ersten Langloch 3 und zu dem gegenüberliegenden Seitenrand der Platte gegen die Mitte seitlich seitlich versetzt erstreckt. Zwischen den beiden Langlöchern 3, 4 ist eine Gewindebohrung 5 vorgesehen. Wie aus Fig, 9 ersichtlich ist, sind auf der Unterseite der Platte die beiden Ränder 6, 7 nach unten hervorstehend ausgebildet derart, daß der zweite Abschnitt 2 zwischen der Unterseite der Platte und den Seitenrändern 6, 7 nach Art einer Schiene geführt ist. An dem einen Ende weist der erste Abschnitt zwei gegen die Mitte jeweils versetzte Bohrungen 8, 9 zur Aufnahme der in Fig. 4 gezeigten Verankerungsschrauben 10 auf. Die Bohrungen sind in bekannter Weise sphärisch angesenkt, so daß die Verankerungsschrauben mit ihrem auf der dem Schaft 11 zugewandten Seite des Kopfes 12 sphärisch ausgebildeten Abschnitt in der Aussenkung liegen und um einen vorbestimmten Winkel verschwenkbar sind.

Der zweite Abschnitt 2 ist ebenfalls als länglich ausgebildetes Rechteck geformt, wobei seine Außenabmessungen so gewählt sind, daß er in der oben beschriebenen schienenförmigen Unterseite des ersten Abschnittes verschiebbar ist. Insbesondere ist also die Breite des plattenförmigen Abschnittes etwas kleiner als der Abstand der Ränder 6, 7 voneinander, und die Länge entspricht im wesentlichen der Länge des schienenförmigen Abschnittes. Der zweite Abschnitt weist eine erste Gewindebohrung 13 auf, die um einen vorbestimmten Wert derart von der Symmetrieachse der Platte zu dem ersten Rand hin versetzt ist, daß der Mittelpunkt der Gewindebohrung 13 mit der Mittenachse des ersten Langloches 3 zusammenfällt, wenn der zweite Abschnitt in den ersten Abschnitt eingeschoben ist. Ferner ist eine zweite Gewindebohrung 14 vorgesehen, die zu dem gegenüberliegenden Seitenrand derart versetzt ist, daß ihr Mittelpunkt mit der Mittenachse des zweiten Langloches 4 zusammenfallt, wenn die beiden Abschnitt ineinandergeschoben sind. Die Anordnung der beiden Gewindebohrungen 13, 14 in Längsrichtung der Platte des zweiten Abschnittes ist so gewählt, daß die beiden Mittelpunkte der Gewindebohrungen 13, 14 in dem in Fig. 2 gezeigten vollständig eingeschoben Zustand mit den Mittelpunkten der jeweiligen den Bohrungen 8, 9 zugewandten kreissegmentförmigen Rändern der Langlöcher 3, 4 zusammenfallen. An dem beim Einschieben den Bohrungen 8, 9 gegenüberliegenden Ende weist der zweite Abschnitt entsprechende Bohrungen 15, 16 zur Aufnahme von Verankerungsschrauben 10 auf.

Die beiden Abschnitte 1, 2 werden dadurch miteinander verbunden, daß in Fig. 5 gezeigte Feststellschrauben von der dem zweiten Abschnitt abgewandten Seite her durch die Langlöcher 3, 4 in die Gewindebohrungen 13, 14 lose eingeschraubt werden. Es ist auf diese Weise eine Relativbewegung der beiden Abschnitte zwischen der in Fig. 2 gezeigten zusammengesteckten Stellung und der in Fig. 3 gezeigten auseinandergezogenen Stellung, in der die Feststellschrauben 17 am anderen Ende der Langlöcher 3, 4 anliegen, möglich.

Wie am besten aus Fig. 1 ersichtlich ist, weist die Platte des zweiten Abschnittes 2 zwischen den beiden Gewindebohrungen 13 und 15 einen eine Struktur aufweisenden Abschnitt 18 auf. In dem gezeigten Ausführungsbeispiel ist die Struktur dadurch gebildet, daß ein sich parallel zu der sich in Längsrichtung erstreckenden Mittenachse der Platte angeordnetes Schraubensegment; wie dieses in den Figuren 7 und 8 am besten zu erkennen ist, geformt ist, dessen Oberfläche sich in Umfangsrichtung des schraubenförmigen Abschnittes erstreckende Rillen aufweist oder das nach Art eines Gewindes oder Kreuzgewindes ausgebildet ist. Die Mittenachse des strukturierten Abschnittes geht, wie aus den Figuren 2 und 3 ersichtlich ist, im eingeschobenen Zustand durch den Mittelpunkt der Gewindebohrung 5 hindurch. Die Länge des strukturierten Abschnittes ist wenigstens gleich der Länge der Langlöcher 3, 4. Seine Lage relativ zu den Langlöchern 3, 4 ist so gewählt, daß die Gewindebohrung 5 von dem in Fig. 2 gezeigten zusammengeschobenen Zustand bis zu dem in Fig. 3 gezeigten maximal auseinandergezogenen Zustand stets mit dem strukturierten Abschnitt 18 deckungsgleich ist.

Zum Einstellen der Relativposition der beiden Abschnitte 1, 2 zueinander ist ein in Fig. 6 gezeigtes Justierwerkzeug 19 vorgesehen. Eine Einrichtung dieser Art ist aus der WO 96/02198 bekannt. Dieses weist ein Gehäuse mit einem zylinderförmigen Mantel 20 und einer Stirnseite 21 auf. In einem an die Stirnseite 21 angrenzenden Bereich des Mantels ist ein Außengewinde 22 vorgesehen, welches dem Innengewinde der Gewindebohrung 5 entspricht. In der Stirnseite des Mantels ist eine Zylinderbohrung 23 mit einem ersten Radius vorgesehen. Angrenzend an den Zylinderabschnitt 23 ist im Inneren des Gehäuses eine koaxiale Bohrung 24 vorgesehen, deren Durchmesser größer als der Durchmesser des Zylinderabschnittes 23 ist. Die Bohrung 24 erstreckt sich bis an das der Stirnseite gegenüberliegende Ende des Mantels, so daß das Gehäuse an diesem Ende offen ist. An diesem der Stirnseite abgewandten Ende des Mantels weist der Mantel ein Innengewinde 25 auf. Außen weist dieses Ende einen sechskantförmigen Querschitt zum Ineingriffgelangen mit einem Schraubschlüssel auf. Das Justierwerkzeug 19 weist ferner ein in dem Gehäuse gehaltenes Druckteil 26 auf, welches in dem Zylinderabschnitt 23 gleitet und an seinem der Stirnseite abgewandten Ende einen der koaxialen Bohrung 24 entsprechenden Durchmesser aufweist, der einen Anschlag für ein maximales Herausfahren in der aus Fig. 6 ersichtlichen Weise bildet. Die Länge des Druckteiles ist so gebildet, daß in der in Fig. 6 gezeigten Anschlagstellung das Druckteil mit seinem freien Ende über die Stirnseite 21 nach außen hervorsteht. Auf der dem hervorstehenden freien Ende abgewandten Seite des Druckteiles ist eine Druckfeder 29 vorgesehen, die mit ihrem anderen Ende an einer in das Innengewinde 25 eingeschraubten Schraube 27 anliegt. An seiner aus der Öffnung des Mantels 20 herausstehenden Stirnseite weist das Druckteil 26 einen ringförmigen Vorsprung 28 auf, dessen Kanten abgerundet sind Der Durchmesser des ringförmigen Vorsprunges entspricht einem ganzzahligen Vielfachen der Teilung des Rundgewindes des strukturierten Abschnittes 18, und die Höhe des ringförmigen Vorsprunges ist kleiner als die Gangtiefe des Rundgewindes, so daß jeweils gegenüberliegende Bereiche des ringförmigen Vorsprunges in die Gewindegänge des strukturförmigen Abschnittes 18 eingreifen können, wenn das Justierwerkzeug 19 in der in Fig. 7 bzw. Fig. 8 gezeigten Weise in die Gewindebohrung 5 eingeschraubt ist.

In jedem der beiden die Bohrungen 8, 9 bzw. 15, 16 aufweisenden Randabschnitte der ersten und zweiten Abschnitte ist eine Gewindebohrung 30 vorgesehen. Diese dient zur Aufnahme einer in Fig. 10 gezeigten Sicherungsschraube 31 mit zugehöriger Sicherungsplatte 32. Die Anordnung der Gewindebohrung 30 relativ zu den Bohrungen 8, 9 bzw. 15, 16 ist so gewählt, daß die Sicherungsplatte bei eingesetzten Verankerungsschrauben 10 gerade über den seitlichen Rand der Verankerungsschraube 10 greift, ohne aber die konzentrische Sechskantbohrung 33 im Kopf der Verankerungsschraube 10, die zum Eingreifen eines Schraubwerkzeuges dient, abzudecken.

Im Betrieb stellt der Operateur die ungefähre Länge der Knochenplatte durch Herausziehen bzw. Hineinschieben des zweiten Abschnittes aus bzw. in den ersten Abschnitt ein und justiert diese Position mit Hilfe des in die Bohrung 5 eingesetzen Justierwerkzeuges. Dann setzt er die Verankerungsschrauben 10. Anschließend erfolgt nun gewünschtenfalls eine Distraktion oder Kontraktion durch Verschieben der beiden Abschnitte 1, 2 unter Einwirkung des Justierwerkzeuges relativ zueinander. Ist die endgültige Einstellung erreicht, werden die zunächst nur lose eingeschraubten Feststellschrauben 17 arretiert. Das bisher erforderliche jeweilige Lösen und Wiedereinsetzen der Verankerungsschrauben 10 und Austauschen der Platten entfällt. Am Ende wird das Justierwerkzeug entfernt und die Sicherungsschrauben 31 festgezogen.

## Patentansprüche

1. Knochenplatte mit einem länglich ausgebildeten Mittelteil und wenigstens einem Loch an jedem Ende zur Aufnahme einer Verankerungsschraube (10), einem eine strukturierte Oberfläche aufweisenden Abschnitt (18) an einem der Abschnitte (2) und einem Fixierelement (19) zum direkten Ineingriffbringen mit der Struktur zum Zwecke der Relativeinstellung der beiden Abschnitte (1, 2) zueinander,
dadurch gekennzeichnet, daß das Mittelteil einen ersten Abschnitt (1) und einen damit zu verbindenden zweiten Abschnitt (2) aufweist, auf dem die strukturierte Oberfläche vorgesehen ist, mit einem sich in Längsrichtung des Mittelteiles erstreckenden Langloch (3, 4) im ersten Abschnitt, einer Bohrung (13, 14) im zweiten Abschnitt (2) und einer die beiden Abschnitte (1, 2) verbindenden, durch das Langloch (3, 4) geführten Schraube (17), und einer mit der strukturierten Oberfläche in Deckung bringbaren Ausnehmung (5) in dem ersten Abschnitt (1) zum lösbaren Ineingriffbringen des Fixierelementes mit der Struktur.

2. Knochenplatte nach Anspruch 1, dadurch gekennzeichnet, daß die Ausnehmung als eine Gewindebohrung (5) zum Aufnehmen des Fixierelementes (19) ausgebildet ist.

3. Knochenplatte nach Anspruch 2, dadurch gekennzeichnet, daß das Fixierelement ein in koaxialer Richtung zu dem Stab federnd vorgespanntes Element aufweist, welches den Stab zu der strukturierten Oberfläche hin auf dem anderen Abschnitt vorspannt.

4. Knochenplatte nach einem der Ansprüche 1 bis 3, dadurch gekennzeichnet, daß der strukturierte Abschnitt eine schraubenförmige Wellung aufweist.

5. Knochenplatte nach Anspruch 4, dadurch gekennzeichnet, daß der zu dem strukturierten Abschnitt hin vorgespannte Teil einen ringförmigen Vorsprung zum Ineingriffbringen mit der strukturierten Oberfläche aufweist.

## Claims

1. Bone plate with an elongated middle part and at least one hole at each end for receiving an anchoring screw (10), with a portion (18) having a structured surface on one of the portions (2) and a locating element (19) for direct engagement with the structure for the purpose of the relative adjustment of the two portions (1, 2) with respect to one another,
characterized in that the middle part has a first portion (1) and a second portion (2), which is to be connected thereto and on which the structured surface is provided, with an oblong hole (3, 4) extending in the longitudinal direction of the middle part in the first portion, a bore (13, 14) in the second portion (2) and a screw (17), which connects the two portions (1, 2) and is passed through the oblong hole (3, 4), and an opening (5) in the first portion (1), which opening can be brought to coincide with the structured surface and is intended for the detachable engagement of the locating element with the structure.

2. Bone plate according to Claim 1, characterized in that the opening is designed as a threaded bore (5) for receiving the locating element (19).

3. Bone plate according to Claim 2, characterized in that the locating element has an element which is resiliently prestressed in the coaxial direction with respect to the bar and prestresses the bar towards the structured surface on the other portion.

4. Bone plate according to one of Claims 1 to 3, characterized in that the structured portion has a helical undulation.

5. Bone plate according to Claim 4, characterized in that the part which is prestressed towards the structured portion has an annular projection for engagement with the structured surface.

## Revendications

1. Plaque pour ostéosynthèse comprenant une partie centrale de forme allongée et au moins un trou sur chaque extrémité destiné à recevoir une vis de fixation (10), une partie (18) présentant une surface structurée, réalisée sur l'une des parties (2) et un élément de blocage (19) destiné à entrer directement en prise avec la surface structurée, afin de pouvoir positionner les deux parties (1, 2) l'une par rapport à l'autre, caractérisée en ce que la partie centrale comporte une première partie (1) et une deuxième partie (2) à assembler avec cette dernière et sur laquelle est prévue la surface structurée, avec un trou oblong (3, 4) réalisé dans la première partie dans le sens longitudinal de la partie centrale, un trou (13, 14) réalisé dans la deuxième partie (2) et une vis (17), guidée à travers le trou oblong pour assembler les deux parties (1, 2), et une cavité (5) réalisée dans la première partie (1) et susceptible de coïncider avec la surface structurée, destinée à fixer de manière amovible l'élément de blocage avec la surface structurée.

2. Plaque pour ostéosynthèse selon la revendication 1, caractérisée en ce que la cavité est conçue sous forme de trou taraudé (5), destiné à recevoir l'élément de blocage (19).

3. Plaque pour ostéosynthèse selon la revendication 2, caractérisée en ce que l'élément de blocage comporte un élément précontraint dans le sens coaxial, de manière flexible par rapport à la tige, lequel élément exerce une précontrainte sur la tige par rapport à la surface structurée réalisée sur l'autre partie.

4. Plaque pour ostéosynthèse selon l'une quelconque des revendications 1 à 3, caractérisée en ce que la partie à surface structurée est formée par une surface ondulée en forme de vis.

5. Plaque pour ostéosynthèse selon la revendication 4, caractérisée en ce que la partie précontrainte par rapport à la partie à surface structurée comporte une saillie annulaire destinée à entrer en prise avec la surface structurée.
